# EUROPÄISCHE PATENTSCHRIFT

(11) **EP 1 898 843 B1**
(45) Veröffentlichungstag und Bekanntmachung des Hinweises auf die Patenterteilung: **27.07.2011**
(21) Anmeldenummer: 06723632.3
(22) Anmeldetag: 22.03.2006
(51) Int. Cl.: A61F 2/44

(54) **BANDSCHEIBENIMPLANTAT**
INTERVERTEBRAL DISK IMPLANT
IMPLANT DE DISQUE INTERVERTEBRALE

(30) Priorität: 03.06.2005 DE 102005025685
(43) Veröffentlichungstag der Anmeldung: 19.03.2008
(73) Patentinhaber: Mathys AG Bettlach, 2544 Bettlach (CH)
(72) Erfinder: DELFOSSE, Daniel, CH-3306 Jegensdorf (CH); SALOMON, Dirk, 04626 Jonaswalde (DE)
(74) Vertreter: Körfer, Thomas
(86) Internationale Anmeldenummer: PCT/EP2006/002640
(87) Internationale Veröffentlichungsnummer: WO 2006/128509

(56) Entgegenhaltungen:
- EP-A- 1 374 807
- EP-A- 1 475 059
- DE-U1- 20 313 183
- FR-A- 2 860 974
- US-A- 5 425 773

## Beschreibung

Die Erfindung geht aus von einem mobilen Bandscheibenimplantat, insbesondere einem Implantat mit Keramik-Keramik-Artikulation.

Beispielsweise ist aus der EP 1 287 794 A1 eine künstliche Bandscheibe bekannt, welche zwei Endplatten und eine elastisch verformbare, zwischen den Endplatten in axialer Richtung unter einer Vorspannung verspannte Scheibe umfaßt. Die Scheibe liegt innerhalb eines rohrförmigen, elastischen Faserrings, wobei die Endplatten mit dem Faserring in zugfester Verbindung stehen. Die Endplatten sind dabei aus Metall gefertigt.

Nachteilig an der aus der EP 1 287 794 A1 bekannten künstlichen Bandscheibe ist insbesondere das schlechte Gleitverhalten. Die Bandscheibe gemäß dem Stand der Technik kann zwar axiale Dehnungen und Stauchungen gut abfangen, bei Verschiebungen zwischen den Wirbeln bzw. insbesondere bei der Neigung der Wirbel gegeneinander kann die Bandscheibe jedoch schlecht reagieren.

Um dies zu umgehen, sollen Keramikimplantate verwendet werden, welche ein besseres Gleitverhalten aufweisen. Diese besitzen jedoch den gravierenden Nachteil, daß im Falle eines Bruchs des relativ spröden Keramikmaterials das Verletzungsrisiko des prothetisch versorgten Patienten sehr hoch ist und gravierende Folgen bis hin zur Querschnittslähmung und zum Tod haben kann, wenn Splitter des Keramikimplantats in den Rückenmarkskanal gelangen.

Der nächstliegende stand des Technik ist in der FR 2 860 974 A1 offenbart. Der Erfindung liegt somit die Aufgabe zugrunde, ein Bandscheibenimplantat anzugeben, welches einerseits ein gutes Gleitverhalten hat und andererseits so gestaltet ist, daß Schadensfälle des Implantats sich nicht bedrohlich auf die Gesundheit des Patienten auswirken können.

Die Aufgabe wird durch die Merkmale des Anspruchs 1 gelöst.

Erfindungsgemäß ist dabei vorgesehen, daß das keramische Bandscheibenimplantat in einer Umhüllung gekapselt ist, welche einerseits fest genug ist, bei einem etwaigen Schadensfall die Bruchstücke des Implantats zusammenzuhalten, und andererseits flexibel genug, um die für die natürliche physiologisch notwendige, uneingeschränkte Beweglichkeit des Implantats zu ermöglichen. Erfindungsgemäß weisen zwei Teile des Umhüllung jeweils einen umlaufenden kragenartigen Überstand auf, wobei sich die kragenartigen Überstände unter Bildung eines Bewegungsspaltes gegenseitig untergreifen.

Weitere vorteilhafte Ausgestaltungen der Erfindung sind in den Unteransprüchen angegeben.

Ausführungsbeispiele der Erfindung werden im folgenden anhand teilweise schematischer Darstellungen in der Zeichnung näher erläutert. In der Zeichnung zeigen:
- Fig.: 1 eine schematische Ansicht eines ersten Ausführungsbeispiels eines erfindungsgemäß ausgestalteten Bandscheibenimplantats in situ,
- Fig. 2: eine vergrößerte Schnittdarstellung des ersten Ausführungsbeispiele des erfindungsgemäßen Bandscheibenimplantats gemäß Fig. 1, und
- Fig. 3: ein zweites Ausführungsbeispiel eines erfindungsgemäßen Bandscheibenimplantats in gleicher Darstellung wie Fig. 2.

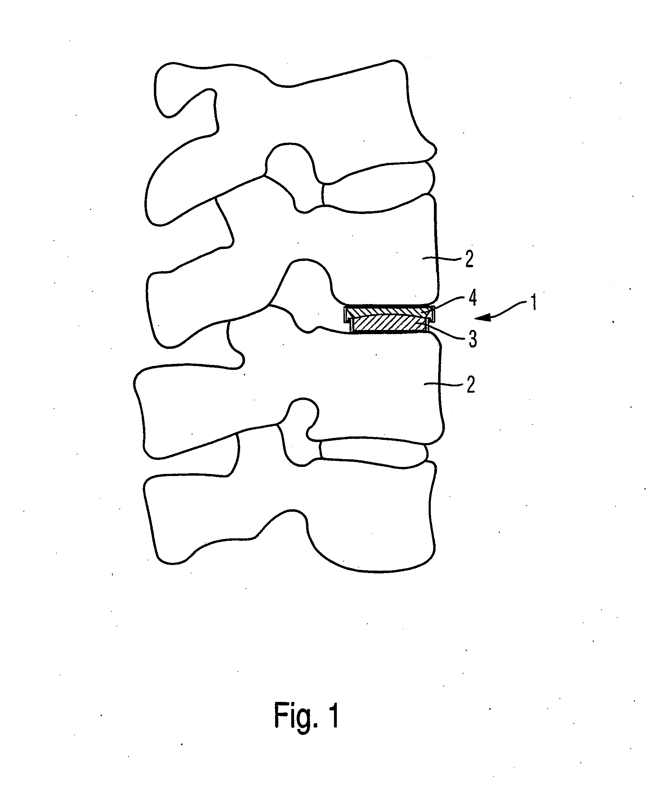

Fig. 1 zeigt in einer stark schematisierten seitlichen Darstellung ein erfindungsgemäß ausgestaltetes Bandscheibenimplantat 1 zwischen zwei Wirbelkörpern 2 einer menschlichen Wirbelsäule.

Das Bandscheibenimplantat 1 muß für einen physiologischen Bewegungsablauf sorgen, wobei insbesondere die Verkippung der im ersten Ausführungsbeispiel zwei das Bandscheibenimplantat bildenden Komponenten 3 und 4 gegeneinander von Bedeutung ist. Hierbei spielt das Gleitverhalten der Komponenten 3 und 4 aufeinander eine entscheidende Rolle. Im Gegensatz zu den bisher üblichen Kunststoffimplantaten, welche defekte Bandscheiben ersetzen, zeigt eine Gleitpaarung zwischen zwei aus Keramik bestehenden Komponenten 3 und 4 ein besseres Gleitverhalten. Insbesondere ist die Haftreibung auch bei hohen Belastungen geringer.

Das Problem bei keramischen Bandscheibenimplantaten 1 ist jedoch das hohe Verletzungsrisiko mit einem hohen Verletzungsgrad bis hin zu Querschnittslähmung und Tod, wenn im Fall eines Bruchs der keramischen Bandscheibenimplantate 1 einzelne Bruchstücke bis zum Rückenmarkskanal vordringen und in diesen mit ihren scharfen Bruchkanten eindringen.

Um dies zu verhindern, wird erfindungsgemäß vorgeschlagen, die beiden keramischen Komponenten 3 und 4 mit einer Umhüllung 5 zu versehen, welche aus einem Kunststoffmaterial, vorzugsweise aus einem biokompatiblen Polymer wie Polyethylen (PE), Polyetheretherketon (PEEK), Polyetherketonketon (PEKK), Polyetheracrylketon (PEAK), Polyacryletherketon (PAEK) oder Polymethylmethacrylat (PMMA) hergestellt ist und das keramische Bandscheibenimplantat 1 allseitig umschließt.

Erfindungsgemaß ist die Umhüllung 5 zweiteilig ausgebildet, um eine leichte Montage vor der Implantation zu gewährleisten. Die beiden Teile 6 und 7 der Umhüllung weisen dabei einen überlappenden Bereich 8 auf, in welchem sich zwei geeignet gestaltete kragenartige Überstände 9 und 10 gegenseitig untergreifen, so daß einerseits ein Bewegungsspalt 11 verbleibt, welcher den gewünschten Bewegungsradius von ca. 10° in vollem Umfang erlaubt, und andererseits die keramischen Komponenten 3 und 4 zuverlässig umschlossen sind.

In der Umhüllung 5, insbesondere in dem Teil 7, welches eine radial größere Abmessung aufweist, kann ein umlaufender Ring 12 vorgesehen sein, welcher eine Stabilisierung der Komponente 4 in dem Teil 7 ermöglicht und als Fixierung bei einer eventuellen Vormontage geeignet ist. Der umlaufende Ring 12 kann als sich nach innen erstreckender Wulst einstückig mit dem Teil 7 ausgebildet oder nachträglich eingebracht und fixiert sein.

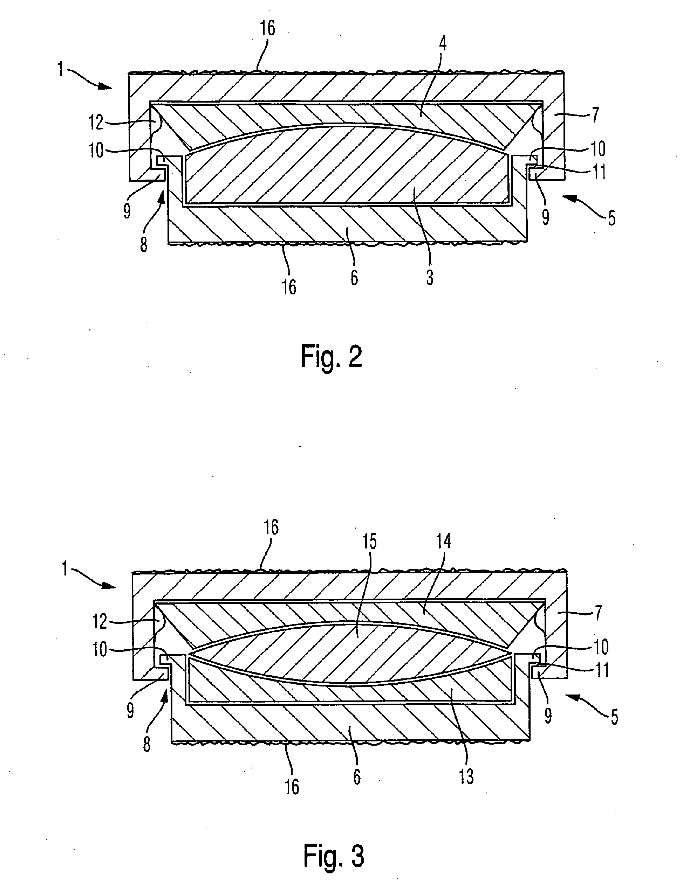

Fig. 3 zeigt in einer schematischen Schnittdarstellung in gleicher Ansicht wie Fig. 2 ein zweites Ausführungsbeispiel eines erfindungsgemäß ausgestalteten. Bandscheibenimplantats 1. Gleiche Bauteile sind dabei mit übereinstimmenden Bezugszeichen versehen.

Das in Fig. 3 dargestellte zweite Ausführungsbeispiel eines erfindungsgemäß ausgestalteten Bandscheibenimplantats 1 ist mit drei keramischen Komponenten 13, 14, 15 versehen, wobei die Komponenten 13 und 14 den in Fig. 2 mit 3 und 4 bezeichneten Komponenten entsprechen. Zwischen den Komponenten 13 und 14 ist eine dritte, ebenfalls keramische, linsenförmige Komponente 15 ausgebildet, welche in den beiden im zweiten Ausführungsbeispiel konkav ausgebildeten Komponenten 13 und 14 eingebettet ist. Ein derartig ausgebildetes Bandscheibenimplantat 1 bietet eine große Beweglichkeit bei ebenfalls geringer Bauhöhe. Die Umhüllung 5 ist wie in dem in Fig. 2 dargestellten ersten Ausführungsbeispiel ausgeführt.

Ein weiterer Vorteil einer erfindungsgemäß ausgestaltet Umhüllung 5 ist die geringe Bauhöhe von nur 4 mm bis 8 mm. Dies ist wichtig, da die keramischen Bandscheibenimplantate 1 insgesamt eine geringe Bauhöhe aufweisen, welche bedingt durch die Einbausituation nicht überschritten werden darf.

Um die Bauteilabmessungen zu minimieren, können deshalb auch faserverstärkte Teile 6, 7, beispielsweise aus kohlefaserverstärktem PEEK, zur Anwendung kommen.

Die Umhüllung 5 kann an ihren den Wirbelkörpern 2 zugewandten Flächen mit einer Titan-Beschichtung 16 versehen sein. Dies führt zu einer Anrauhung der Flächen und zu einer verbesserten Osseo-Integration.

Die Erfindung ist nicht auf die dargestellten Ausführungsbeispiele beschränkt und auch für weitere Ausführungsformen von Bandscheibenimplantaten 1 geeignet. Alle Merkmale der Erfindung sind beliebig miteinander kombinierbar.

## Patentansprüche

1. Bandscheibenimplantat (1) zum Implantieren zwischen zwei Wirbelkörpern (2); umfassend zumindest zwei Komponenten (3, 4; 13, 14, 15), welche zumindest eine Gleitpaarung bilden und welche aus einem keramischen Material gefertigt sind,
wobei die zumindest zwei keramischen Komponenten (3, 4; 13, 14, 15) in einer Umhüllung (5) angeordnet sind
**dadurch gekennzeichnet,**
**dass** zwei Teile (6, 7) der Umhüllung (5) jeweils einen umlaufenden kragenartigen Überstand (9, 10) aufweisen und dass sich die kragenartigen überstände (9, 10) gegenseitig unter Bildung eines Bewegungsspalts (11) untergreifen.

2. Bandscheibenimplantat nach Anspruch 1,
**dadurch gekennzeichnet,**
**dass** die Umhüllung (5) aus zwei Teilen besteht.

3. Bandscheibenimplantat nach Anspruch 2,
**dadurch gekennzeichnet,**
**dass** die Teile (6, 7) der Umhüllung (5) lösbar miteinander verbindbar sind.

4. Bandscheibenimplantat nach Anspruch 1 oder 3,
**dadurch gekennzeichnet;**
**dass** in einem oder beiden der Teile (6, 7) der Umhüllung (5) ein umlaufender Ring (12) ausgebildet ist.

5. Bandscheibenimplantat nach Anspruch 4,
**dadurch.gekennzeichnet,**
**dass** die in dem jeweiligen Teil (6, 7) der Umhüllung (5) einliegende Komponente (3, 4; 13, 14) des Bandscheibenimplantats (1) durch den Ring (12) fixiert ist.

6. Bandscheibenimplantat nach Anspruch 4 oder 5,
**dadurch gekennzeichnet,**
**dass** der Ring (12) als Wulst einstückig mit dem jeweiligen Teil (6, 7) ausgebildet ist.

7. Bandscheibenimplantat nach Anspruch 4 oder 5,
**dadurch gekennzeichnet,**
**dass** der Ring (12) als separates, mit dem jeweiligen Teil (6, 7) verbindbare Komponente ausgebildet ist.

8. Bandscheibenimplantat nach einem der Ansprüche 1 bis 7,
**dadurch gekennzeichnet,**
**dass** die Umhüllung (5) aus einem biokompatiblen Kunststoffmaterial besteht.

9. Bandscheibenimplantat nach Anspruch 8,
**dadurch gekennzeichnet,**
**dass** der Kunststoff ein Polymer, insbesondere Polyethylen (PE), Polyetheretherketon (PEEK), Polyetherketonketon (PEKK), Polyetheracrylketon (PEAK), Polyacryletherketon (PAEK) oder Polymethylmethacrylat (PMMA), ist.

10. Bandscheibenimplantat nach einem der Ansprüche 1 bis
**dadurch gekennzeichnet,**
**dass** die Umhüllung (5) an ihren den Wirbelkörpern (2) zugewandten Flächen mit einer Beschichtung (16) versehen ist.

11. Bandscheibenimplantat nach Anspruch 10,
**dadurch gekennzeichnet,**
**dass** die Beschichtung (16) aus Titan besteht.

12. Bandscheibenimplantat nach einem der Ansprüche 1 bis 11,
**dadurch gekennzeichnet,**
**dass** die Umhüllung (5) des Bandscheibenimplantats (1) eine axiale Höhe von 4 mm bis 8 mm aufweist.

13. Bandscheibenimplantat nach einem der Ansprüche 1 bis 12,
**dadurch gekennzeichnet,**
**dass** die Umhüllung (5) faserverstärkt ist.

14. Bandscheibenimplantat nach Anspruch 13,
**dadurch gekennzeichnet,**
**dass** die Faserverstärkung aus Kohlefaser besteht.

## Claims

1. An intervertebral disc implant (1) for implanting between two vertebrae (2), comprising at least two components (3, 4; 13, 14, 15), which form at least one sliding pair and which are made from a ceramic material, the at least two ceramic components (3, 4; 13, 14, 15) being arranged in a casing (5),
**characterised in that**
two parts (6, 7) of the casing (5) each comprise a circumferential collar-like projection (9, 10) and **in that** the collar-like projections (9, 10) engage one under the other, forming a movement gap (11).

2. An intervertebral disc implant according to claim 1,
**characterised in that**
the casing (5) consists of two parts.

3. An intervertebral disc implant according to claim 2,
**characterised in that**
the parts (6, 7) of the casing (5) may be connected detachably together.

4. An intervertebral disc implant according to claim 1 or claim 3,
**characterised in that**
a circumferential ring (12) is formed in one or both of the parts (6, 7) of the casing (5).

5. An intervertebral disc implant according to claim 4,
**characterised in that**
the component (3, 4; 13, 14) of the intervertebral disc implant (1) enclosed in the respective part (6, 7) of the casing (5) is fixed in place by the ring (12).

6. An intervertebral disc implant according to claim 4 or claim 5,
**characterised in that**
the ring (12) takes the form of a bead in one piece with the respective part (6, 7).

7. An intervertebral disc implant according to claim 4 or claim 5, **characterised in that**
the ring (12) takes the form of a separate component connectable to the respective part (6, 7).

8. An intervertebral disc implant according to one of claims 1 to 7,
**characterised in that**
the casing (5) consists of a biocompatible plastics material.

9. An intervertebral disc implant according to claim 8,
**characterised in that**
the plastics material is a polymer, in particular polyethylene (PE), polyetheretherketone (PEEK), polyetherketoneketone (PEKK), polyetheracrylketone (PEAK), polyacryletherketone (PAEK) or polymethyl methacrylate (PMMA).

10. An intervertebral disc implant according to one of claims 1 to 9,
**characterised in that**
the casing (5) is provided with a coating (16) on its faces facing the vertebrae (2).

11. An intervertebral disc implant according to claim 10,
**characterised in that**
the coating (16) consists of titanium.

12. An intervertebral disc implant according to one of claims 1 to 11,
**characterised in that**
the casing (5) of the intervertebral disc implant (1) has an axial height of 4 mm to 8 mm.

13. An intervertebral disc implant according to one of claims 1 to 12,
**characterised in that**
the casing (5) is fibre-reinforced.

14. An intervertebral disc implant according to claim 13,
**characterised in that**
the fibre reinforcement consists of carbon fibre.

## Revendications

1. Implant de disque intervertébral (1) destiné à être implanté entre deux vertèbres (2) et comprenant au moins deux composants (3, 4 ; 13, 14, 15) qui forment au moins un couple de glissement et sont fabriqués en un matériau céramique,
lesdits au moins deux composants céramiques (3, 4; 13, 14, 15) étant agencés dans une enveloppe (5), **caractérisé**
**en ce que** deux parties (6, 7) de l'enveloppe (5) présentent chacune un bord périphérique en saillie (9, 10) à la manière d'une collerette, et en ce que les bords en saillie (9, 10) en forme de collerette s'engagent réciproquement l'un sous l'autre en ménageant un interstice de mouvement (11).

2. Implant de disque intervertébral selon la revendication 1,
**caractérisé**
**en ce que** l'enveloppe (5) est constituée de deux parties.

3. Implant de disque intervertébral selon la revendication 2,
**caractérisé**
**en ce que** les parties (6, 7) de l'enveloppe (5) peuvent être reliées l'une à l'autre de manière amovible.

4. Implant de disque intervertébral selon la revendication 1 ou la revendication 3,
**caractérisé**
**en ce que** dans l'une ou les deux parties (6, 7) de l'enveloppe (5), est formé un anneau périphérique (12).

5. Implant de disque intervertébral selon la revendication 4,
**caractérisé**
**en ce que** le composant (3, 4; 13, 14) de l'implant de disque intervertébral (1), placé dans la partie respective (6, 7) de l'enveloppe (5), est fixé par ledit anneau (12).

6. Implant de disque intervertébral selon la revendication 4 ou la revendication 5,
**caractérisé**
**en ce que** l'anneau (12) est réalisé sous forme de bourrelet d'un seul tenant avec la partie (6, 7) respective.

7. Implant de disque intervertébral selon la revendication 4 ou la revendication 5,
**caractérisé**
**en ce que** l'anneau (12) est réalisé sous forme de composant séparé pouvant être relié à la partie (6, 7) respective.

8. Implant de disque intervertébral selon l'une des revendications 1 à 7,
**caractérisé**
**en ce que** l'enveloppe (5) est réalisée en une matière plastique biocompatible.

9. Implant de disque intervertébral selon la revendication 8,
**caractérisé**
**en ce que** la matière plastique est un polymère, notamment du polyéthylène (PE), polyétheréthercétone (PEEK), polyéthercétonecétone (PEKK) polyéther-acrylcétone (PEAK), polyacryléthercétone (PAEK) ou polyméthacrylate de méthyle (PMMA).

10. Implant de disque intervertébral selon l'une des revendications 1 à 9,
**caractérisé**
**en ce que** l'enveloppe (5) est pourvue d'un revêtement (16) sur ses surfaces dirigées vers les vertèbres (2).

11. Implant de disque intervertébral selon la revendication 10,
**caractérisé**
**en ce que** le revêtement (16) est réalisé en titane.

12. Implant de disque intervertébral selon l'une des revendications 1 à 11,
**caractérisé**
**en ce que** l'enveloppe (5) de l'implant de disque intervertébral (1) présente une hauteur axiale de 4 mm à 8 mm.

13. Implant de disque intervertébral selon l'une des revendications 1 à 12,
**caractérisé**
**en ce que** l'enveloppe (5) est renforcée de fibres.

14. Implant de disque intervertébral selon la revendication 13,
**caractérisé**
**en ce que** le renforcement de fibres est réalisé en fibres de carbone.
